# EUROPEAN PATENT SPECIFICATION

(11) **EP 0 661 998 B1**
(45) Date of publication and mention of the grant of the patent: **25.11.1998**
(21) Application number: 93905520.8
(22) Date of filing: 05.03.1993
(51) Int. Cl.: A61K 39/04, A61K 35/74

(54) **THERAPEUTIC AGENT FROM MYCOBACTERIUM VACCAE AND ITS USE AGAINST HIV INFECTION WITH AIDS**
THERAPEUTISCHES AGENZ AUS MYCOBACTERIUM VACCAE UND SEINE VERWENDUNG GEGEN EINE HIV-INFEKTION MIT AIDS
AGENT THERAPEUTIQUE OBTENUE A PARTIR DE MYCOBACTERIUM VACCAE ET SON UTILISATION POUR LUTTER CONTRE L'INFECTION HIV AVEC SIDA

(30) Priority: 14.09.1992 GB 9219425
(43) Date of publication of application: 12.07.1995
(73) Proprietor: UNIVERSITY COLLEGE LONDON, London WC1E 6HA (GB)
(72) Inventor: STANFORD, John, Lawson Mill House Claygate, Kent TN12 9TE (GB); ROOK, Graham, Arthur, William Old Hall, Haverhill Suffolk CB9 7EJ (GB)
(74) Representative: Cresswell, Thomas Anthony
(86) International application number: GB9300463
(87) International publication number: WO9406466

(56) References cited:
- WO-A-91/01751
- REVIEWS OF INFECTIOUS DISEASES vol. 11, no. 2, March 1989, CHICAGO IL, US pages 349 - 351 G. K[LLENIUS ET AL. 'Does vaccination with Bacille Calmette-Guérin protect against AIDS?'
- THE LANCET vol. 338, no. 8781, 14 December 1991, LONDON, GB pages 1533 - 1534 A. POZNIAK ET AL. 'Mycobacterium vaccae immunotherapy.'

## Description

This invention relates to therapeutic agents useful in the therapy of HIV infection with AIDS (acquired immune deficiency syndrome).

The causative agent for AIDS is known to be a virus of the retrovirus family called HIV (human immunodeficiency virus). Infection with HIV does not, however, immediately give rise to overt symptoms of AIDS. The only indication of exposure to the virus may be the presence of antibodies thereto in the blood of an infected subject who is then described as 'HIV positive'. The infection may lie dormant, giving rise to no obvious symptoms, and the incubation period prior to development of AIDS may vary from several months to decades. Development of AIDS itself may be preceded by the AIDS-related complex (ARC) which is characterised by unexplained fever, weight loss, chronic cough or diarrhoea.

The reasons for the variable period between infection with the virus and breakdown of the immune system in an infected individual are poorly understood. Factors at present unknown may trigger proliferation of the virus with consequential disruption of she immune system. The victims of the disease are then subject to various infections and malignancies which, unchecked by the disabled immune system, lead to death.

The association between HIV infection and tuberculosis is well known. An early effect of HIV infection is the reactivation of previously dormant tubercule bacilli. The maintenance of resistance to mycobacteria is an active immunological process which is compromised by HIV infection. In dually infected persons, there is a high reactivation rate of dormant tubercule bacilli and this reactivation usually occurs well before the appearance of other HIV/AIDS-related infections which strongly suggests that an important effect of HIV infection is to destroy precisely those immune functions, presumably T-cell mediated, that maintain mycobacterial dormancy.

There is also evidence that where active tuberculosis is superimposed on HIV infection, there is a dramatic loss of CD4 T-cells which results in very rapid development of overt symptoms of AIDS. It appears in fact that immune mediators released in tuberculosis accelerate transactivation of the HIV provirus.

We have previously described the use of antigenic and/or immuno regulatory material derived from Mycobacterium vaccae in the treatment of tuberculosis (see, for example, British Patent No. 2156673 and United States Patent No. 4724144). In our International Patent Application No. PCT/GB90/01169 (publication No. WO91/01751), we have described the use of the same material for immunoprophylactic treatment against AIDS, i.e. for increasing the period between infection by HIV and development of AIDS.

We have now discovered that the same therapeutic agent not only delays development of AIDS in patients infected by HIV, but also is capable of causing regression, or even removal, of overt symptoms of AIDS even in patients where the disease is far advanced. These effects have been found in patients suffering also from tuberculosis, but are expected to occur also in patients who are suffering from HIV infection with or without AIDS and without associated tuberculosis.

The present invention accordingly provides the use of antigenic and/or immunoregulatory materials derived from Mycobacterium vaccae for the manufacture of a medicament useful in the therapy of AIDS, with or without associated tuberculosis. Such material may be administered to a subject, for example a subject who is HIV positive and shows overt symptoms of AIDS with or without infection by Mycobacterium tuberculosis, in an amount sufficient at least to arrest the progression of the symptoms.

The therapeutic agent conveniently, and therefore preferably, comprises dead cells of M. vaccae, most preferably cells which have been killed by autoclaving. The immunotherapeutic agent normally comprises more than 10⁸ microorganisms per ml of diluent, and preferably from 10⁸ to 10¹¹ killed M. vaccae microorganisms per ml of diluent.

The diluent may be pyrogen-free saline for injection alone, or a borate buffer of pH 8.0. The diluent should be sterile. A suitable borate buffer is:
- Na₂B₄0₇.10H₂0: 3.63 g
- H₃BO₃: 5.25 g
- NaCl: 6.19 g
- Tween: 0.0005%
- Distilled Water: to 1 litre

The preferred strain of M. vaccae is one denoted R877R isolated from mud samples from the Lango district of Central Uganda (J.L. Stanford and R.C. Paul, Ann. Soc. Belge Med, Trop. 1973, 53, 141-389). The strain is a stable rough variant and belongs to the aurum sub-species. It can be identified as belonging to M. vaccae by biochemical and antigenic criteria (R. Bonicke, S.E. Juhasz., Zentr albl. Bakteriol. Parasitenkd. Infection skr. Hyg. Abt. 1, Orig., 1964, 192, 133).

The strain denoted R877R has been deposited at the National Collection of Type Cultures (NCTC) Central Public Health Laboratory, Colindale Avenue, London NW9 5HT, United Kingdom on February 13th, 1984 under the number NCTC 11659.

For the preparation of the immunotherapeutic agent, the microorganism M. vaccae may be grown on a suitable solid medium. A modified Sauton's liquid medium is preferred (S.V. Boyden and E. Sorkin., J. Immunol, 1955 75, 15) solidified with agar.

Preferably the solid medium contains 1.3% agar. The medium inoculated with the microorganisms is incubated aerobically to enable growth of the microorganisms to take place, generally at 32°C for 10 days. The organisms are harvested, then weighed and suspended in a diluent. The diluent may be unbuffered saline but is preferably borate-buffered and contains a surfactant such as Tween 80 as described above. The suspension is diluted to give 100 mg of microorganism/ml. For further dilution, borate buffered saline is preferably used so that the suspension contains 10 mg wet weight of microorganisms/ml of diluent. The suspension may then be dispensed into 5 ml multidose vials. Although the microorganisms in the vials may be killed using irradiation e.g. from ⁶⁰Cobalt at a dose of 2.5 megarads, or by any other means, for example chemically, it is preferred to kill the microorganisms by autoclaving, for example at 10 psi for 10 minutes (115°-125°C). It has been discovered that autoclaving yields a more effective preparation than irradiation.

The immunotherapeutic agent is in general administered by injection in a volume in the range 0.1-0.2 ml, preferably 0.1 ml, given intradermally. A single dosage will generally contain from 10⁷ to 10¹⁰ killed M. vaccae microorganisms. It is preferred to administer to patients a single dose containing 10⁸ to 10⁹ killed M. vaccae. However, the dose may be repeated depending on the condition of the patient.

Although the immunotherapeutic agent will generally be administered by intradermal injection, other routes, e.g. oral administration, can also be used.

HIV infection with AIDS, including AIDS associated with tuberculosis may be treated by a method which comprises administering to a subject suffering from HIV infection with AIDS, and with or without associated tuberculosis, antigenic and/or immunoregulatory material derived from Mycobacterium vaccae in an amount sufficient to provoke an immune response effective against the AIDS symptoms.

For 20 to 50% of African patients with HIV infection tuberculosis is the first symptom in development of AIDS. Among a group of patients being treated for tuberculosis were seventeen who were seropositive by the Wellcome ELISA for HIV1. All the patients were prescribed streptomycin, isoniazid, rifampicin and pyrazinamide for their tuberculosis. Therapy was abbreviated and did not last longer than three months in any case. Eight of the seventeen patients received the therapeutic agent of the present invention and nine received placebo (saline). At follow up about one year later only three of the patients who had received the anti-tuberculosis drugs only had survived and all three of these had advanced tuberculosis. Seven of the eight patients treated with the therapeutic agent of the present invention had become sputum smear negative for acid fast bacilli (i.e. tubercule bacilli) and the general improvement in their condition was similar to that in tuberculosis patients who were not HIV positive. Five of the eight patients had generalised lymphadenopathy at the time of diagnosis. This had resolved at the time of follow-up. The only two patients who were retested serologically at the follow-up were found to be negative for HIV1.

It may be advantageous and is within the scope of the invention to use more than one strain of M. vaccae, and/or to include in the therapeutic agent other mycobacterial antigens. Tuberculin may also be included.

The therapeutic agent may also contain BCG (Bacillus Calmette-Guerin) vaccine, in particular the freeze-dried form of the vaccine, to promote its effect.

The therapeutic agent can contain further ingredients such as adjuvants, preservatives, stabilisers etc. It may be supplied in sterile injectable liquid form or in sterile freeze-dried form which is reconstituted prior to use.

M. vaccae may be used as such or as an extract or fractionated portion of the organism to prepare therapeutic agents according to the invention.

The following Example describes the preparation of a therapeutic agent as used in the invention.

### EXAMPLE

M. vaccae is grown on a solid medium comprising modified Sauton's medium solidified with 1.3% agar. The medium is inoculated with the microorganism and incubated for 10 days at 32°C to enable growth of the microorganism to take place. The microorganisms are then harvested and weighed and suspended in diluent to give 100 mg of microorganisms/ml of diluent. The suspension is then further diluted with buffered saline to give a suspension containing 10 mg wet weight of microorganisms/ml of diluent and dispensed into 5 ml multidose vials. The vials containing the live microorganism are then autoclaved for 10 minutes at 10 psi to kill the microorganisms and give the immunotherapeutic agent of the invention, which may (if desired) be further diluted for use.

This immunotherapeutic agent may be administered by intradermal injection in the manner already described.

## Claims

1. Use of antigenic and/or immunoregulatory material derived from Mycobacterium vaccae for the manufacture of a medicament useful in the therapy of HIV infection with AIDS.

2. The use according to claim 1, wherein the antigenic and/or immunoregulatory material derived from M. vaccae comprises dead cells of M. vaccae.

3. The use according to claim 2, wherein the cells of M. vaccae have been killed by autoclaving.

4. The use according to any one of the preceding claims wherein the material derived from M. vaccae is derived from the strain as deposited at the National Collection of Type Cultures (NCTC) Central Public Health Laboratory, Colindale Avenue, London, NW9 5HT, United Kingdom on February 13th, 1984 under the number NCTC 11659.

5. The use according to any one of the preceding claims wherein the material derived from M. vaccae is contained in a medicament comprising from 10⁷ to 10¹⁰ microorganisms per dose.

6. The use according to any one of the preceding claims wherein the HIV infection with AIDS is associated with tuberculosis.

## Patentansprüche

1. Verwendung eines von Mycobacterium vaccae abgeleiteten antigenen und/oder immunoregulatorischen Materials zur Herstellung eines Medikamentes zur Therapie einer HIV-Infektion mit AIDS.

2. Verwendung nach Anspruch 1, dadurch gekennzeichnet, daß das von M. vaccae abgeleitete antigene und/oder immunoregulatorische Material tote Zellen von M. vaccae umfaßt.

3. Verwendung nach Anspruch 3, dadurch gekennzeichnet, daß die Zellen von M. vaccae durch Autoklavieren abgetötet wurden.

4. Verwendung nach einem der vorhergehenden Ansprüche, dadurch gekennzeichnet, daß das von M. vaccae abgeleitete Material abgeleitet ist von dem beim National Collection of Type Cultures (NCTC) Central Public Health Laboratory, Colindale Avenue, London, NW9 5HT, United Kingdom, am 13. Februar 1984 unter der Nummer NCTC 11659 hinterlegten Stamm.

5. Verwendung nach einem der vorhergehenden Ansprüche, dadurch gekennzeichnet, daß das von M. vaccae abgeleitete Material in einem Medikament enthalten ist, das 10⁷ bis 10¹⁰ Mikroorganismen pro Verabreichungsdosis enthält.

6. Verwendung nach einem der vorhergehenden Ansprüche, dadurch gekennzeichnet, daß die HIV-Infektion mit AIDS mit Tuberkulose assoziiert ist.

## Revendications

1. Utilisation d'un matériau antigénique et/ou immunorégulateur dérivé de Mycobacterium vaccae pour la préparation d'un médicament utile dans le traitement de l'infection par le VIH avec SIDA.

2. Utilisation selon la revendication 1, dans laquelle le matériau antigénique et/ou immunorégulateur dérivé de M. vaccae comprend des cellules de M. vaccae mortes.

3. Utilisation selon la revendication 2, dans laquelle les cellules de M. vaccae ont été tuées par passage à l'autoclave.

4. Utilisation selon l'une quelconque des revendications précédentes, dans laquelle le matériau dérivé de M. vaccae est dérivé de la souche telle que déposée auprès de la National Collection of Type Cultures (NCTC) Central Public Health Laboratory, Colindale Avenue, Londres, NW9 5HT, Grande Bretagne le 13 février 1984, sous le numéro NCTC 11659.

5. Utilisation selon l'une quelconque des revendications précédentes, dans laquelle le matériau dérivé de M. vaccae est contenu dans un médicament comprenant de 10⁷ à 10¹⁰ micro-organismes par dose.

6. Utilisation selon l'une quelconque des revendications précédentes, dans laquelle l'infection par le VIH avec SIDA est associée à la tuberculose.
